(19) Europäisches Patentamt
European Patent Office
Office européen des brevets

(11) Publication number: **0 260 697 B1**

(12) **EUROPEAN PATENT SPECIFICATION**

(45) Date of publication of patent specification: **17.03.93** (51) Int. Cl.5: **A61K 7/06**

(21) Application number: **87113632.1**

(22) Date of filing: **17.09.87**

The file contains technical information submitted after the application was filed and not included in this specification

(54) **Composition for application to hair.**

(30) Priority: **18.09.86 JP 220211/86**
**28.04.87 JP 105230/87**

(43) Date of publication of application:
**23.03.88 Bulletin 88/12**

(45) Publication of the grant of the patent:
**17.03.93 Bulletin 93/11**

(84) Designated Contracting States:
**AT BE CH DE ES FR GB IT LI NL SE**

(56) References cited:
**EP-A- 0 129 778**
**EP-A- 0 256 472**
**DE-A- 2 139 183**
**FR-A- 2 077 725**
**FR-A- 2 590 169**
**GB-A- 2 151 924**

**W.P.I.L., FILE SUPPLIER, 8th October 1971, abstract no. 71-64589S, Derwent Publications Ltd., London, GB; & JP-B-46 034 440 (KYOWA HAKKO KOGYO CO.)**

(73) Proprietor: **LION CORPORATION**
**3-7, Honjo 1-chome**
**Sumida-ku Tokyo(JP)**

(72) Inventor: **Sugiyama, Keikichi**
**18-13, Ishigamidai 1-chome Oiso-machi**
**Naka-gun Kanagawa-ken(JP)**
Inventor: **Takada, Koji**
**909-2, Daigiri**
**Fujisawa-shi Kanagawa-ken(JP)**
Inventor: **Yamamoto, Ikuo**
**Copo-Gurasu 402 585-24, Renshoji**
**Odawara-shi Kanagawa-ken(JP)**

(74) Representative: **KUHNEN, WACKER & PARTNER**
**Alois-Steinecker-Strasse 22 Postfach 1553**
**W-8050 Freising (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

### BACKGROUND OF THE INVENTION

#### Field of the Invention

The present invention relates to a composition for application to hair in the manner of a hair tonic, which, upon application to the scalp, activates melanocytes of the radix pili and improves synthesis of melanin, thereby preventing graying of the hair and restoring grayed hair to its natural color.

### DESCRIPTION OF THE PRIOR ART

Graying of the hair is a universal aging phenomena, while hair dyes are commonly used to dye grayed hair, the use of such dyes is troublesome and sometimes causes side-effects such as rash. Therefore, many users find hair dyes to be an unsatisfactory solution. There has thus been a great need for developing a pharmaceutical agent or a cosmetic composition for hair capable of essentially preventing graying of the hair and restoring graying hair to its natural color.

Heretofore, there have been developed various kinds of drugs for activating the radix pili and promoting the growth of hair. On the other hand, there have been reported only a few cosmetic compositions for preventing graying of the hair and restoring grayed hair to its natural color (see, for instance, Japanese Patent Un-examined Publication (KOKAI) Nos.60-174705, 61-165310, 62-45527, 62-63509 and 62-63510). Moreover, the reported techniques suffer from various disadvantages such as low effectiveness, low stability of the active components and insufficient safety.

The composition disclosed in Japanese Patent Unexamined Publication No. 60-174705 employs vitamin $D_3$ and derivatives thereof. However, the cosmetic agent is incomplete in the following points:

(i) It is difficult to form the vitamin $D_3$ and its derivatives into cosmetic preparations since they are extremely unstable in the air and easily undergo oxidation, so that the cosmetic agent cannot be expected to provide sufficient effect when applied as external preparation;

(ii) Vitamin $D_3$ and the derivatives thereof tend to cause side-effects such as hypervitaminosis D or hypercalcemia (anorexia, vomiting, constipation, disponderal, degrowth and the like) and, therefore, there has been some doubt about safety.

Consequently, the cosmetic compositions disclosed on the above-mentioned Un-examined Publications are of little practical use.

GB-A-2151924 discloses a cosmetic or dermatological composition for skin care which comprises oenothera oil and extract of spleen tissue and optionally cAMP besides further components.

DE-A-2139183 discloses cosmetical and pharmaceutical compositions showing skin activity, which comprise cAMP as active substance.

FR-A-2077725 discloses pharmaceutical compositions against the ageing of the skin, the hair and the nails comprising cAMP as active ingredient.

None of these documents discloses or suggests that cAMP and other nucleic acid related substances have influence on the graying of the hair.

### SUMMARY OF THE INVENTION

Many people are annoyed by their grayed hair and there is a great need for developing compositions for preventing graying of the hair or restoring grayed hair to its natural color without causing side-effects such as those mentioned above.

Accordingly, it is a principal object of the present invention to provide a method for preventing graying of the hair or restoring grayed hair to its natural color upon application of an effective amount of a solution to the scalp which causes no side-effects and has high safety.

The present invention has been completed on the basis of the discovery that the aforementioned problems can effectively be eliminated by using a specific nucleic acid related material and derivatives thereof, which are present in various tissues and cells of the living organisms in trace amounts, and have various regulatory and physiological effects.

According to the present invention , the foregoing and other objects can be effectively accomplished by providing a composition for application to hair (or the scalp) which comprises at least one component (A) (nucleic acid related substance) selected from the group consisting of compounds having the following general formula (I), and salts thereof:

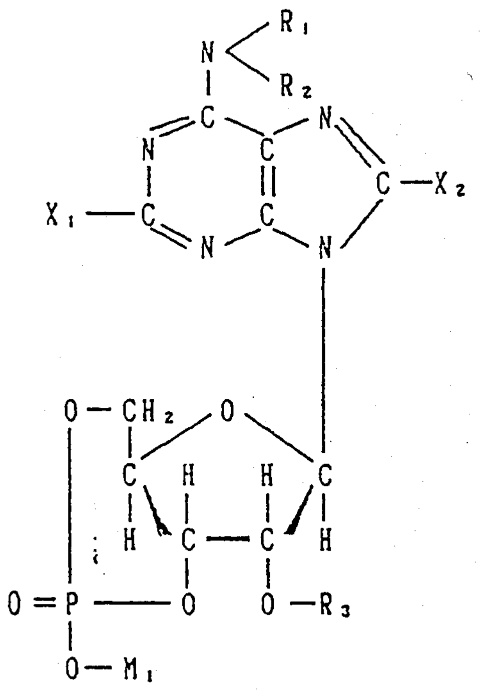

(I)

In the general formula (I), $R_1$, $R_2$ and $R_3$ may be the same or different and each represents hydrogen atom, an acyl group having 1 to 25, preferably 1 to 19 carbon atoms or an alkyl group having 1 to 25, preferably 1 to 19 carbon atoms; $X_1$ and $X_2$ respectively represent hydrogen atom; a halogen atom such as fluorine, chlorine, bromine or iodine; a mercapto group, a thioalkyl group having 1 to 4 carbon atoms, 4-chlorophenylthio group and thiobenzyl group; amino group; an aminoalkyl group having 1 to 4 carbon atoms; or hydroxyl group; and $M_1$ represents hydrogen atom or a salt-forming cation such as an alkali metal (e.g., sodium, potassium or lithium) and tris(hydroxymethyl)aminomethane. In this respect, the acyl and alkyl groups may each have a substituent such as a halogen atom and may further include an aromatic ring. Moreover, the acyl group may be one derived from a dibasic acid.

## DESCRIPTION OF THE PREFERRED EMBODIMENTS

Examples of the compounds represented by the general formula (I) include adenosine 3',5'-cyclic phosphoric acid (compound of the formula (I) in which all of $R_1$, $R_2$, $R_3$, $X_1$, $X_2$ and $M_1$ are hydrogen atoms; hereunder referred to as cAMP), the specific derivatives and the salts thereof.

These compounds, and the salts thereof will hereinafter be explained in more detail.

Compounds Represented by the General Formula (I):

cAMP as used herein is a physiologically important material which exerts regulatory function on various effects of hormones in the living organisms and it has practically been used as a biochemical reagent and medicine effective for cardiac diseases. cAMP has been produced using fermentation techniques and synthetic methods on an industrial scale. Moreover, a variety of derivatives thereof which are improved in cell membrane permeability or in stability within the living organisms are commercially available.

Specific examples of compounds (I) include those listed in the following Table I and sodium, potassium and lithium salts thereof which may be incorporated into the composition of the present invention alone or in a combination. In Table I and hereinafter, the abbreviation ClPhS denotes 4-chlorophenylthio group.

Table I

| Groups | $R_1$ | $R_2$ | $R_3$ | $X_1$ | $X_2$ |
|---|---|---|---|---|---|
| 1(cAMP) | H | H | H | H | H |
| 2 | acetyl | H | H | H | H |
| | butyryl | H | H | H | H |
| | hexanoyl | H | H | H | H |
| | octanoyl | H | H | H | H |
| | lauroyl | H | H | H | H |
| | pentadecanoyl | H | H | H | H |
| | malonyl | H | H | H | H |
| | succinyl | H | H | H | H |
| | benzoyl | H | H | H | H |
| | methyl | H | H | H | H |
| | ethyl | H | H | H | H |
| | ethyl | ethyl | H | H | H |
| 3 | acetyl | H | H | amino | H |
| | acetyl | H | H | aminomethyl | H |
| | butyryl | H | H | Br | H |
| | butyryl | H | H | ClPhS | H |
| | succinyl | H | H | Br | H |
| | succinyl | H | H | ClPhS | H |
| | ethyl | H | H | Cl | H |
| | ethyl | H | H | mercapto | H |

Table I (continued)

| Groups | $R_1$ | $R_2$ | $R_3$ | $X_1$ | $X_2$ |
|---|---|---|---|---|---|
| 4 | acetyl | H | H | H | amino |
| | acetyl | H | H | H | aminomethyl |
| | butyryl | H | H | H | Br |
| | butyryl | H | H | H | ClPhS |
| | succinyl | H | H | H | Br |
| | succinyl | H | H | H | ClPhS |
| | ethyl | H | H | H | Cl |
| | ethyl | H | H | H | mercapto |
| 5 | H | H | acetyl | H | H |
| | H | H | butyryl | H | H |
| | H | H | hexanoyl | H | H |
| | H | H | octanoyl | H | H |
| | H | H | lauroyl | H | H |
| | H | H | pentadecanoyl | H | H |
| | H | H | malonyl | H | H |
| | H | H | succinyl | H | H |
| | H | H | benzoyl | H | H |
| | H | H | methyl | H | H |
| | H | H | ethyl | H | H |

Table I (continued)

| Groups | $R_1$ | $R_2$ | $R_3$ | $X_1$ | $X_2$ |
|---|---|---|---|---|---|
| 6 | H | H | acetyl | amino | H |
| | H | H | acetyl | aminomethyl | H |
| | H | H | butyryl | Br | H |
| | H | H | butyryl | ClPhS | H |
| | H | H | succinyl | Br | H |
| | H | H | succinyl | ClPhS | H |
| | H | H | ethyl | Cl | H |
| | H | H | ethyl | mercapto | H |
| 7 | H | H | acetyl | H | amino |
| | H | H | acetyl | H | aminomethyl |
| | H | H | butyryl | H | Br |
| | H | H | butryl | H | ClPhS |
| | H | H | succinyl | H | Br |
| | H | H | succinyl | H | ClPhS |
| | H | H | ethyl | H | Cl |
| | H | H | ethyl | H | mercapto |
| 8 | H | H | H | Br | H |
| | H | H | H | Cl | H |
| | H | H | H | mercapto | H |
| | H | H | H | thiomethyl | H |
| | H | H | H | ClPhS | H |

Table I (continued)

| Groups | $R_1$ | $R_2$ | $R_3$ | $X_1$ | $X_2$ |
|---|---|---|---|---|---|
| | H | H | H | amino | H |
| | H | H | H | aminomethyl | H |
| | H | H | H | hydroxyl | H |
| 9 | H | H | H | H | Br |
| | H | H | H | H | Cl |
| | H | H | H | H | mercapto |
| | H | H | H | H | thiomethyl |
| | H | H | H | H | ClPhS |
| | H | H | H | H | amino |
| | H | H | H | H | aminomethyl |
| | H | H | H | H | hydroxyl |
| 10 | H | H | H | Br | Br |
| | H | H | H | Cl | Cl |
| | H | H | H | mercapto | mercapto |
| | H | H | H | thiomethyl | thiomethyl |
| | H | H | H | ClPhS | ClPhS |
| | H | H | H | amino | amino |
| | H | H | H | aminomethyl | aminomethyl |
| | H | H | H | hydroxyl | hydroxyl |

Table I (continued)

| Groups | $R_1$ | $R_2$ | $R_3$ | $X_1$ | $X_2$ |
|---|---|---|---|---|---|
| 11 | acetyl | H | acetyl | H | H |
| | butyryl | H | butyryl | H | H |
| | hexanoyl | H | hexanoyl | H | H |
| | octanoyl | H | octanoyl | H | H |
| | lauroyl | H | lauroyl | H | H |
| | pentadecanoyl | H | pentadecanoyl | H | H |
| | malonyl | H | malonyl | H | H |
| | succinyl | H | succinyl | H | H |
| | benzoyl | H | benzoyl | H | H |
| | methyl | H | methyl | H | H |
| | ethyl | H | ethyl | H | H |
| 12 | acetyl | H | acetyl | amino | H |
| | acetyl | H | acetyl | aminomethyl | H |
| | butyryl | H | butyryl | Br | H |
| | butyryl | H | butyryl | ClPhS | H |
| | succinyl | H | succinyl | Br | H |
| | succinyl | H | succinyl | ClPhS | H |
| | ethyl | H | ethyl | Cl | H |
| | ethyl | H | ethyl | mercapto | H |

Table I (continued)

| Groups | $R_1$ | $R_2$ | $R_3$ | $X_1$ | $X_2$ |
|---|---|---|---|---|---|
| 13 | acetyl | H | acetyl | H | amino |
| | acetyl | H | acetyl | H | aminomethyl |
| | butyryl | H | butyryl | H | Br |
| | butyryl | H | butyryl | H | ClPhS |
| | succinyl | H | succinyl | H | Br |
| | succinyl | H | succinyl | H | ClPhS |
| | ethyl | H | ethyl | H | Cl |
| | ethyl | H | ethyl | H | mercapto |
| 14 | acetyl | H | H | amino | amino |
| | acetyl | H | H | aminomethyl | aminomethyl |
| | butyryl | H | H | Br | Br |
| | butyryl | H | H | ClPhS | ClPhS |
| | succinyl | H | H | Br | Br |
| | succinyl | H | H | ClPhS | ClPhS |
| | ethyl | H | H | Cl | Cl |
| | ethyl | H | H | mercapto | mercapto |
| 15 | H | H | acetyl | amino | amino |
| | H | H | acetyl | aminomethyl | aminomethyl |
| | H | H | butyryl | Br | Br |
| | H | H | butyryl | ClPhS | ClPhS |
| | H | H | succinyl | Br | Br |

Table I (continued)

| Groups | $R_1$ | $R_2$ | $R_3$ | $X_1$ | $X_2$ |
|---|---|---|---|---|---|
| | H | H | succinyl | ClPhS | ClPhS |
| | H | H | ethyl | Cl | Cl |
| | H | H | ethyl | mercapto | mercapto |
| 16 | acetyl | H | acetyl | amino | amino |
| | acetyl | H | acetyl | aminomethyl | aminomethyl |
| | butyryl | H | butyryl | Br | Br |
| | butyryl | H | butyryl | ClPhS | ClPhS |
| | succinyl | H | succinyl | Br | Br |
| | succinyl | H | succinyl | ClPhS | ClPhS |
| | ethyl | H | ethyl | Cl | Cl |
| | ethyl | H | ethyl | mercapto | mercapto |

Among these compounds, it is preferable to use the compound in which at least one of $R_1$, $R_2$, $R_3$, $X_1$ and $X_2$ is not a hydrogen atom in the general formula (I). Furthermore, it is preferable to use the compound in which $R_1$ and $R_3$ each is a hydrogen atom or an acyl group, $R_2$ and $X_1$ each is hydrogen atom, and $X_2$ is a hydrogen atom, a halogen atom, a sulfur-containing group, an amino group or a hydroxyl group. In case where $X_2$ is a hydrogen atom and both or one of $R_1$ and $R_3$ is an acyl group in these compounds, it is more preferable that the acyl group derived from a monocarboxylic acid have 5 to 15 carbon atoms, and the acyl group derived from a dicarboxylic acid have 2 to 15 carbon atoms. In case where $X_2$ is a halogen atom, a sulfur-containing group, an amino group or a hydroxyl group, it is also more preferable that $R_1$ and $R_2$ each be a hydrogen atom or an acyl group having 1 to 15 carbon atoms.

The foregoing nucleic acid related materials (component (A)) used as an essential component in the composition for hair are directly applied to the scalp thereby providing a greatly improved effect of preventing graying of the hair and restoring grayed hair to its natural color hair. Such an excellent effect has never been recognized before. These materials may be incorporated into compositions for application to hair in any concentration. Although, the amount thereof to be incorporated generally varies depending on the form of the preparation and frequency of application, it is desirable to use these compounds in an amount of 0.001 to 5% by weight (hereafter referred to as %), preferably 0.01 to 2% in various compositions.

In addition to the foregoing components, the composition of this invention may further comprise other pharmaceutical components such vitamins as vitamin A, vitamin $B_6$, vitamin E, pantothenic acid and biotin; such amino acids as methionine, cysteine, cystine and tyrosine; such antibacterial agents as salicylic acid, hinokitiol, resorcin and trichlorocarbanilide; and such hormones as ethynylestradiol and progesterone and these pharmaceutical components are preferably added to compositions for hair in an amount of 0.0001 to 3%.

Moreover, other materials commonly used in cosmetics can also be added to the composition for hair of the present invention and examples thereof include oils, water, surfactants, humectants, lower alcohols, thickening agents, antioxidants, chelating agents, pH-adjusting agents, preservatives, perfumes and color

additives. Examples of oils include fats and oils such as olive oil, jojoba oil and hardened oil; waxes such as spermaceti, beeswax and lanolin; hydrocarbons such as liquid paraffin, ceresin and squalane; fatty acids such as stearic acid and oleic acid; alcohols such as cetanol, stearyl alcohol, lanolin alcohol and hexyl decanol; and esters such as isopropyl myristate and butyl stearate. These oils are incorporated into the composition for hair alone or in combination and the amount thereof ranges from 0.5 to 85%.

Examples of surfactants are anionic surfactants such as sodium stearate, sodium cetylsulfate, polyoxyethylene laurylether phosphate and sodium N-acyl glutamate; cationic surfactants such as stearyldimethylbenzylammonium chloride and stearyltrimethylammonium chloride; amphoteric surfactants such as alkylaminoethylglycine hydrochloride solutions and lecithin; and nonionic surfactants such as glycerin monostearate, sorbitan monostearate, sucrose fatty acid esters, propylene glycol monostearate, polyoxyethylene oleylether, polyethylene glycol monostearate, polyoxyethylene sorbitan monopalmitate, polyoxyethylene coconut fatty acid monoethanolamide, polyoxyethylene polyoxypropylene glycol, polyoxyethylene castor oil and polyoxyethylene lanolin. These surfactants may be used in the composition for hair alone or in combination and the amount thereof falls within the range of 0.1 to 15%.

Moreover, there may be mentioned such humectants as glycerin, 1,3-butylene glycol and propylene glycol; such lower alcohols as ethanol and isopropanol; such thickening agents as polyethylene glycol and sodium carboxymethylcellulose; such antioxidants as dibutylhydroxytoluene, butylhydroxyanisole and propyl gallate; such chelating agents as disodium edetate and ethanehydroxy diphosphate; such pH-adjusting agents as citric acid, sodium citrate, boric acid, borax and disodium hydrogen phosphate; and such preservatives as methyl para-hydroxybenzoate, ethyl para-hydroxybenzoate, dehydroacetic acid, salicylic acid and benzoic acid.

In this respect, these optional components are not restricted to those specific examples listed above.

The compositions for hair of the present invention may be prepared by appropriately admixing the foregoing essential components and optional components and may be used in any forms capable of external use such as hair tonics, creams, lotions, milky lotions and ointments. For instance, hair tonics comprise 0.01 to 2% of the essential components (component (A)) of the present invention, 40 to 98% of a lower alcohol, 0 to 3% of the foregoing pharmaceutical component, 0 to 15% of a humectant, 0 to 60% of purified water and a small amount of a perfume; or comprise 0.01 to 2% of the essential component (A), 40 to 98% of a lower alcohol, 0 to 3% of the foregoing pharmaceutical component, 0 to 15% of a humectant, 0 to 60% of purified water and a small amount of a perfume; hair creams comprise 0.01 to 2% of the essential component (A) of the present invention, 20 to 80% of an oil, 0.5 to 15% of a surfactant, 0 to 15% of a humectant, 15 to 80% of purified water and a small amount of a preservative; or comprise 0.01 to 2% of the essential component (A), 20 to 80% of an oil, 0.5 to 15% of a surfactant, 0 to 15% of a humectant, 15 to 80% of purified water and a small amount of a preservative; and milky lotions comprise 0.01 to 2% of the essential component (A) of the present invention, 5 to 30% of an oil, 0.5 to 15% of a surfactant, 0 to 15% of a humectant, 50 to 95% of purified water and a small amount of a preservative; or comprise 0.01 to 2% of the essential component (A), 5 to 30% of an oil, 0.5 to 15% of a surfactant, 0 to 15% of a humectant, 50 to 95% of purified water and a small amount of a preservative.

Although, the reason why the nucleic acid related substances (component (A)) exhibit such an excellent effect of preventing graying of the hair and restoring grayed hair to its natural color has not yet been clearly evidenced, it is assumed that, when externally applied, the melanocytes present in the bulbus pili of the scalp radix pili are activated to promote the synthesis of melanin and the resultant melanin granules are promoted to be incorporated into mother cells of the hair.

The aforementioned nucleic acid related substances as used herein are naturally present in a wide range of the living organisms and, therefore, are considered to be highly safe. When the level of safety was checked in order to make sure, no practical problem was observed with respect to acute toxicity, skin irritation or skin sensitization and thus a high level of safety was confirmed.

In accordance with the present invention, a composition for hair capable of preventing graying of the hair and restoring grayed hair to its natural color upon externally applied to the scalp is provided. Besides, the composition is highly safe as regards the possibility of causing skin damage.

Therefore, the compositon according to the present invention can be widely used in various forms capable of being externally applied. Such forms include, for example, cosmetic compositions such as hair tonics, creams, lotions, milky lotions, hair treatments, hair conditioners; and ointments.

The present invention will hereunder be explained in more detail with reference to the following working examples. Moreover, the effects practically attained according to the invention will also be discussed with reference to comparative examples.

The abbreviations given below are used in the examples.

MB: monobutyryl

MH: monohexanoyl
MO: monooctanoyl
MS: monosuccinyl
DA: diacetyl
DB: dibutyryl
DM: dimalonyl
DS: disuccinyl
clPhS: 4-chlorophenylthio

In the examples, the position of the substituent of the cAMP derivatives is shown as follows:

$N^6$: Binding position of $R_1$ and $R_2$ is indicated except that $R_1$ or $R_2$ is not a hydrogen atom.
$O^{2'}$: Binding position of $R_3$ is indicated except that $R_3$ is not a hydrogen atom.
8: Binding position of $X_2$ is indicated except that $X_2$ is not a hydrogen atom.

Example 1

A perfume was uniformly dissolved in ethanol. In addition, a solution was prepared by uniformly dissolving other components listed below in purified water. Then, the ethanolic solution was added to the aqueous solution to form a hair tonic. Details are shown in Table II wherein each numerical value represents loadings expressed as % by weight (the same in all tables hereafter).

Table II

| Component | Present Invention | Comparative Example |
|---|---|---|
| Ethanol | 80.0 | 80.0 |
| Glycerin | 0.5 | 0.5 |
| $N^6,O^{2'}$-DBcAMP-Na | 0.05 | -- |
| Color Additive | small amount | small amount |
| Perfume | ditto | ditto |
| Purified Water | 19.45 | 19.5 |

The effectiveness of the hair tonics thus prepared were estimated in the following manner:

Twenty statistically equivalent persons (comprising men and women aged 30 to 60 years) were selected and Samples of the hair tonics were applied to their scalps in accordance with the Half-Head technique, in which Samples were separately applied to the right and left half portions of the scalp, twice a day (in the morning and at night) for three months. The effects of preventing graying of the hair and restoring grayed hair to its natural color were estimated by comparing the conditions of the portions observed before and after the application of Samples. The results obtained are summarized in the following Table III.

Table III

| This Invention is better | This Invention is somewhat better | Same | Comp. Sample is somewhat better | Comp. Sample is better |
|---|---|---|---|---|
| 11 | 7 | 2 | 0 | 0 |

As seen from the results shown in Table III, it was found that the hair tonic of the invention comprising $N^6,O^{2'}$-DBcAMP-Na reduced the amount of grayed hair more effectively than the comparative hair tonic free from such component and that the former showed remarkable effect of preventing graying of the hair and restoring grayed hair to its natural color.

No abnormality was observed in the condition of the scalp during and after the use of the hair tonic of the invention for 3 months. Safety tests on $N^6,O^{2'}$-DBcAMP-Na were conducted. The results observed are summarized in Table IV.

Table IV

| Items Examined | Results Observed |
|---|---|
| Acute Oral Toxicity (rat) | $LD_{50}$ = 5 g/kg or more |
| Skin Irritating Property | |
| 1) Primary Skin Irritation (guinea pig) | 5%: non-irritant |
| 2) Phototoxicity (guinea pig) | negative |
| 3) Ocular-Mucous Membrane Irritation (rabbit) | 5%: non-irritant |
| Skin Sensitizing Property | |
| 1) Skin Sensitization (guinea pig) | negative |
| 2) Photosensitization (guinea pig) | negative |
| Human Patch Test | 5%: non-irritant |

The results in Table IV show that the safety of $N^6,O^{2'}$-DBcAMP-Na is extremely high.

Example 2

Hair creams were prepared by separately dissolving components 1 to 6 and components 7 to 10 listed in Table V while heating at 80 °C, then mixing and emulsifying these solutions, adding component 11 during cooling and uniformly dispersing the mixture.

Table V

| Components | Present Invention | Comp. Sample |
|---|---|---|
| 1. Lanolin | 2.5 | 2.5 |
| 2. Sorbitan Monostearate | 5.0 | 5.0 |
| 3. Polyoxyethylene Sorbitan Monostearate (EO $\bar{P}$=20)* | 2.0 | 2.0 |
| 4. Beeswax | 10.0 | 10.0 |
| 5. Liquid Paraffin | 22.0 | 22.0 |
| 6. Hardened Oil | 23.0 | 23.0 |
| 7. Ethyl Parahydroxybenzoate | 0.2 | 0.2 |
| 8. Borax | 0.5 | 0.5 |
| 9. $N^6,O^{2'}$-DScAMP-Na | 0.1 | -- |
| 10. Purified Water | 34.2 | 34.3 |
| 11. Perfume | 0.5 | 0.5 |

*Average number of ethylene oxide adducted (hereinafter referred to as EO $\bar{P}$).

According to the procedures disclosed in Example 1, these hair creams were examined on the effects of preventing graying of the hair and restoring grayed hair to its natural color. Results are summarized in Table VI.

Table VI

| This Invention is better | This Invention is somewhat better | Same | Comp. Sample is somewhat better | Comp. Sample is better |
|---|---|---|---|---|
| 10 | 8 | 2 | 0 | 0 |

As seen from the results listed in Table VI, the hair cream of the present invention containing $N^6,O^{2'}$-DScAMP-Na exhibits an excellent effect of preventing graying of the hair and restoring gray hair to its natural color as compared with the comparative hair cream free from the component and thus the amount of grayed hair was reduced.

In addition, during and after the use of the hair cream of the invention for 3 months, no abnormality was observed in the condition of the scalp. According to the same procedures as in Example 1, the safety of $N^6,O^{2'}$-DScAMP-Na was examined and it was confirmed that it had no problems such as skin irritation and thus had extremely high safety.

Example 3

Milky lotions were prepared by separately dissolving components 1 to 4 and components 5 to 9 listed in Table VII and then adding the solution of the components 1 to 4 to that of the components 5 to 9 with stirring.

Table VII

| Components | Present Invention | Comp. Sample |
|---|---|---|
| 1. Lanolin Alcohol | 5.0 | 5.0 |
| 2. Isopropyl Myristate | 2.0 | 2.0 |
| 3. Stearic Acid | 5.0 | 5.0 |
| 4. Glycerin Monostearate | 1.0 | 1.0 |
| 5. Triethanolamine | 1.0 | 1.0 |
| 6. Propylene Glycol | 5.0 | 5.0 |
| 7. $O^{2'}$-MScAMP-Na | 0.2 | -- |
| 8. Preservative | small amount | small amount |
| 9. Purified Water | 80.8 | 81.0 |

According to the procedures described in Example 1, these lotions were examined on the effects of preventing graying of the hair and restoring grayed hair to its natural color. Results are summarized in Table VIII.

Table VIII

| This Invention is better | This Invention is somewhat better | Same | Comp. Sample is somewhat better | Comp. Sample is better |
|---|---|---|---|---|
| 10 | 7 | 3 | 0 | 0 |

As seen from the results listed in Table VIII, the milky lotion of the present invention containing $O^{2'}$-MScAMP-Na exhibits an excellent effect of preventing graying of the hair and restoring grayed hair to its natural color as compared with the comparative milky lotion free from the component and thus the amount of grayed hair was reduced.

In addition, during and after the use of the milky lotion of the invention for 3 months, no abnormality was observed in the condition of the scalp. According to the same procedures as in Example 1, the safety of $O^{2'}$-MScAMP-Na was examined and it was confirmed that it had no problems such as skin irritation and thus had extremely high safety.

Example 4

Hair creams having composition shown in Table IX were prepared and the effect of preventing graying of the hair and restoring grayed hair to its natural color was examined. Results observed are listed in Table IX.

Table IX

| Components | Present Invention | Comp. Sample |
|---|---|---|
| Cetanol | 25.0 | 25.0 |
| White Vaseline | 25.0 | 25.0 |
| Polyethyleneglycol Monostearate (EO $\bar{P}$=40) | 5.0 | 5.0 |
| Propylene Glycol | 12.0 | 12.0 |
| $N^6$-MBcAMP-Na | 0.8 | -- |
| Preservative | small amount | small amount |
| Purified Water | 32.2 | 33.0 |

Table X

| This Invention is better | This Invention is somewhat better | Same | Comp. Sample is somewhat better | Comp. Sample is better |
|---|---|---|---|---|
| 9 | 7 | 4 | 0 | 0 |

As seen from the results listed in Table X, the hair cream of the present invention containing $N^6$-MBcAMP-Na exhibits an excellent effect of preventing graying of the hair and restoring grayed hair to its natural color as compared with the comparative hair cream free from the component and thus the amount of grayed hair was reduced.

In addition, during and after the use of the hair cream of the invention for 3 months, no abnormality was observed in the condition of the scalp. According to the same procedures as in Example 1, the safety of $N^6$-MBcAMP-Na was examined and it was confirmed that it had no problems such as skin irritation and thus had extremely high safety.

Example 5

The procedures of Example 2 were repeated except that 8-BrcAMP-Na, 8-ClcAMP-Na, 8-ClPhScAMP-Na, 8-SHcAMP-Na, 8-OHcAMP-Na, $O^{2'}$-MB-8-BrcAMP-Na or $N^6$,$O^{2'}$-DA-8-$NH_2$cAMP-Na was used instead of $N^6$,$O^{2'}$-DScAMP-Na to form hair creams and the aforesaid effect was also estimated according to the same manner as in Example 1. Substantially the same excellent effect as in Example 2 was observed.

Example 6

Components shown in Table XI other than ethanol were in order dissolved in ethanol to form hair tonics (Samples S-I to S-III). Composition of each Sample was shown in Table XI.

Table XI

| Component | Present Invention S-I | Present Invention S-II | Comp. Example S-III |
|---|---|---|---|
| Ethanol | 97.45 | 99.45 | 99.5 |
| Glycerin | 0.5 | 0.5 | 0.5 |
| $N^6,O^{2'}$-DBcAMP-Na | 0.05 | 0.05 | -- |
| Glyceryl Monopentadecanoate | 2.0 | -- | -- |
| Color Additive | small amount | small amount | small amount |
| Perfume | ditto | ditto | ditto |

Two groups each of which comprised 20 persons (50 to 60 years old; comprising men and women) were selected and the hair tonic Samples (S-I and S-II) were separately applied to scalp of each person of one group in accordance with Half-Head technique wherein the hair tonics I and II were separately applied to the right half of the scalp and the left half thereof, on the other hand Samples (S-II and S-III) were likewise separately applied to each person of the other group in the same manner twice a day (in the morning and at night) for 3 months. Then, the effects of preventing graying of the hair and restoring grayed hair to its natural color were estimated by comparing the conditions of the portions observed before and after the application of Samples and the results obtained were listed in Tables XII and XIII.

Table XII

| This Invention (S-II) is better | This Invention (S-II) is somewhat better | Same | Comp. Sample (S-III) is somewhat better | Comp. Sample (S-III) is better |
|---|---|---|---|---|
| 7 | 8 | 5 | 0 | 0 |

Table XIII

| This Invention (S-I) is better | This Invention (S-I) is somewhat better | Same | This Invention (S-II) is somewhat better | This Invention (S-II) is better |
|---|---|---|---|---|
| 3 | 8 | 7 | 2 | 0 |

It is confirmed that Samples S-I and S-II according to the present invention are excellent in the effects of preventing graying of the hair and restoring grayed hair to its natural color compared with the comparative example (Sample S-III). This is the very effects due to the incorporation of $N^6,O^{2'}$-DBcAMP-Na or the combination of it with glyceryl monopentadecanoate into the hair tonics. Moreover, the effect of S-I is superior to that of S-II.

In addition, during and after the application of the hair tonics of the invention for 3 months, no abnormality was observed in the condition of the scalp.

Example 7

Hair creams (H-I to H-III) were prepared by separately dissolving components 1 to 6 and components 7 to 10 listed in Table XIV while heating at 80 °C, then admixing and emulsifying these solutions, adding component 11 thereto during cooling and uniformly dispersing the mixture.

Table XIV

| Components | Present Invention (H-I) | Present Invention (H-II) | Comp. Example (H-III) |
|---|---|---|---|
| 1. Beeswax | 10.0 | 10.0 | 10.0 |
| 2. Spermaceti | 5.0 | 5.0 | 5.0 |
| 3. Hardened Oil | 19.5 | 19.5 | 19.5 |
| 4. Liquid Paraffin | 35.0 | 35.0 | 35.0 |
| 5. Antioxidant | small amount | small amount | small amount |
| 6. Glyceryl Triheptadecanoate | 1.5 | -- | -- |
| 7. cAMP-Na | 0.1 | 0.1 | -- |
| 8. Borax | 0.5 | 0.5 | 0.5 |
| 9. Preservative | small amount | small amount | small amount |
| 10. Purified Water | 28.4 | 29.9 | 30.0 |
| 11. Perfume | small amount | small amount | small amount |

According to the same procedures as those in Example 6, these hair creams were examined on the effect of preventing graying of the hair and restoring grayed hair to its natural color and results obtained were listed in Tables XV and XVI.

Table XV

| This Invention (H-II) is better | This Invention (H-II) is somewhat better | Same | Comp. Sample (H-III) is somewhat better | Comp. Sample (H-III) is better |
|---|---|---|---|---|
| 6 | 7 | 7 | 0 | 0 |

Table XVI

| This Invention (H-I) is better | This Invention (H-I) is somewhat better | Same | This Invention (H-II) is somewhat better | This Invention (H-II) is better |
|---|---|---|---|---|
| 4 | 7 | 6 | 3 | 0 |

It is confirmed that Samples H-I and H-II according to the present invention are excellent in the effects of preventing graying of the hair and restoring grayed hair to its natural color compared with the comparative example (Sample H-III) as seen from the results on Tables XV and XVI. This is the very effects due to the incorporation of cAMP-Na or the combination of it with glyceryl triheptadecanoate into the hair creams. Moreover, the effect of H-I is superior to that of H-II.

In addition, during and after the application of the hair creams of the invention for 3 months, no abnormality was observed in the condition of the scalp.

Example 8

Components shown in Table XVII other than ethanol were in order dissolved in ethanol to form Samples I to III (hereunder referred to as S-1 to S-3) of hair tonic. The composition of each Sample is shown in Table XVII.

Table XVII

| Component | Present Invention S-1 | Present Invention S-2 | Comp. Example S-3 |
|---|---|---|---|
| Ethanol | 86.5 | 89.5 | 89.6 |
| Castor Oil | 10.0 | 10.0 | 10.0 |
| Hinokitiol | 0.1 | 0.1 | 0.1 |
| Alpha-tocopherol | 0.3 | 0.3 | 0.3 |
| $N^6,O^{2'}$-DScAMP-Na | 0.1 | 0.1 | -- |
| Pentadecyl Alcohol | 3.0 | -- | -- |
| Color Additive | small amount | small amount | small amount |
| Perfume | ditto | ditto | ditto |

According to Example 6, the hair tonics were examined on the effect of preventing graying of the hair and restoring graying hair to its natural color and results obtained are listed in Tables XVIII and XIX.

Table XVIII

| This Invention (S-2) is better | This Invention (S-2) is somewhat better | Same | Comp. Sample (S-3) is somewhat better | Comp. Sample (S-3) is better |
|---|---|---|---|---|
| 8 | 7 | 5 | 0 | 0 |

Table XIX

| This Invention (S-1) is better | This Invention (S-1) is somewhat better | Same | This Invention (S-2) is somewhat better | This Invention (S-2) is better |
|---|---|---|---|---|
| 5 | 6 | 6 | 3 | 0 |

It is confirmed that Samples S-1 and S-2 according to the present invention are excellent in the effects of preventing graying of the hair and restoring grayed hair to its natural color compared with the comparative example (Sample S-3) as seen from the results in Tables XVIII and XIX. This is the very effects due to the incorporation of $N^6$,$O^{2'}$-DScAMP-Na or the combination of it with pentadecyl alcohol into the hair tonics. Moreover, the effect of S-1 is superior to that of S-2.

In addition, during and after the application of the hair tonics of the invention for 3 months, no abnormality was observed in the condition of the scalp.

Example 9

The procedures of Example 7 were repeated except that cAMP-Na used therein was replaced with $O^{2'}$-MBcAMP-Na to obtain hair creams and they were likewise examined on the aforementioned effect. As a result, approximately the same results as those in Example 7 were obtained.

Example 10

The procedures in Example 7 were repeated except that glyceryl triheptadecanoate was replaced with triundecyl glycerylether or nonanoic acid. The resulting hair creams were examined on the aforesaid effects and approximately the same results as in Example 7 were obtained.

Example 11 (Comparative Example)

Components shown in Table XXIV other than ethanol were in order dissolved in ethanol to form hair tonics. The composition of each hair tonic is shown in Table XXIV.

Table XXIV

| | Example 11 I | Example 11 II |
|---|---|---|
| Ethanol | 75.0 | 75.0 |
| Olive Oil | 0.5 | 0.5 |
| Pyrrolidone Carboxylic Acid | 0.5 | 0.5 |
| FAD-2Na | 0.1 | -- |
| Perfume | small amount | small amount |
| Purified Water | 23.9 | 24.0 |

According to Example 1, the hair tonics were examined on the effect of preventing graying of the hair and restoring grayed hair to its natural color and results obtained are listed in Table XXV.

Table XXV

| Ex. 11 I is better | Ex. 11 I is somewhat better | Same | Ex. 11 II is somewhat better | Ex. 11 II is better |
|---|---|---|---|---|
| 8 | 8 | 4 | 0 | 0 |

Example 12

The procedures in Example 11 were repeated except that $N^6$-MScAMP-Na, $N^6$-MHcAMP-Na, $O^{2'}$-MOcAMP-Na or $N^6,O^{2'}$-DMcAMP-Na was used in place of FAD-2Na used in Example 11 to form hair tonics and the properties thereof were estimated in accordance with the same manner as in Example 1. Thus, it is confirmed that these hair tonics are excellent in the effects of preventing graying of the hair and restoring grayed hairs to its natural color.

Example 13

The procedures of Example 8 were repeated except that glyceryl ditridecanoate, diacetylglyceryl monopentadecanoate, sodium nonadecanoate, methyl heptadecanoate, tridecanoylamide, N-acetylundecanoylamide, N,N-diacetylnonanoylamide, 1,13-tridecamethylenedicarboxylic acid, choresterol nonanoate, 1,2-diundecanoyl-glycero-3-phosphorylcoline, 1,2-dipentadecanoyl-glycero-3-phosphoric acid or N-tridecanoylsphingosine-1-phosphorylethanolamine was used instead of pentadecyl alcohol and 8-clPhScAMP-Na was used instead of $N^6,O^{2'}$-DScAMP-Na to form hair tonics, and the aforesaid effect was also estimated according to the same manner as in Example 8.

As a result, substantially the same excellent effect as in Example 8 was obtained.

## Claims

**1.** Use of nucleic acid related substance selected from the group consisting of compounds having the following general formula (I) and salts thereof for the manufacture of a composition for the prevention of graying of the hair or restoration of grayed hair to its natural color upon application to the scalp:

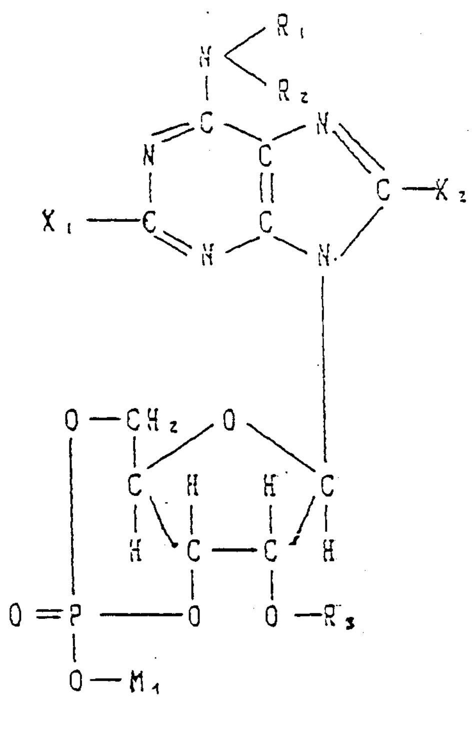

(I)

in the general formula (I), $R_1$, $R_2$ and $R_3$ may be the same or different and each represents hydrogen atom, an acyl group having 1 to 25 carbon atoms, which may have a halogen atom and which further may include an aromatic ring, or an alkyl group having 1 to 25 carbon atoms, which may have a halogen atom and which further may include an aromatic ring; $X_1$ and $X_2$, respectively, represent hydrogen atom, a halogen, mercapto group, a thioalkyl group having 1 to 4 carbon atoms, 4-chlorophenylthio group, thiobenzyl group, amino group, an aminoalkyl group having 1 to 4 carbon atoms or hydroxyl group; and $M_1$ represents hydrogen atom or a saltforming cation.

2. Use as set forth in claim 1 wherein the compound represented by the general formula (I) is adenosine 3', 5'-cyclic-phophoric acid or a salt thereof.

3. A method for preventing graying of the hair or restoring grayed hair to its natural color which comprises applying to the scalp an effective amount of solution comprising the nucleic acid related substance of claim 1.

4. A method as set forth in claim 3 wherein the compound represented by the general formula (I) is adenosine 3',5'-cyclic-phosphoric acid or a salt thereof.

5. A method as set forth in claim 3 and/or 4 wherein the effective amount of the nucleic acid related substances wherein at least one of $R_1$, $R_2$, $R_3$, $X_1$ and $X_2$ is not hydrogen is 0.01 to 2%.

6. A method as set forth in any of claims 3 to 5 wherein the composition is a hair tonic comprising 0.01 to 2% by weight of the nucleic acid related substance, 40 to 98% by weight of a lower alcohol, 0 to 3% by weight of pharmaceutical agents, 0 to 15% by weight of a humectant, 0 to 60% by weight of purified water and a small amount of a perfume.

7. A method as set forth in any of claims 3 to 5 wherein the composition is a hair cream comprising 0.01 to 2% by weight of the nucleic acid related substance, 20 to 80% by weight of an oil, 0.5 to 15% by weight of a surfactant, 0 to 15% by weight of a humectant, 15 to 80% by weight of purified water and a small amount of a preservative.

8. A method as set forth in any claims 3 to 5 wherein the composition is a milky lotion comprising 0.01 to 2% by weight of the nucleic acid related substance, 5 to 30% by weight of an oil, 0.5 to 15% by weight of a surfactant, 0 to 15% by weight of a humectant, 50 to 95 % by weight of purified water and a small amount of a preservative.

**Patentansprüche**

1. Verwendung einer mit Nucleinsäure verwandten Substanz, ausgewählt aus der Gruppe, die aus Verbindungen besteht, die die folgende allgemeine Formel (I) aufweisen und deren Salze, für die Herstellung einer Zusammensetzung zur Verhinderung des Ergrauens des Haars oder zur Wiederherstellung von ergrautem Haar zu seiner natürlichen Farbe nach Anwendung auf die Kopfhaut;

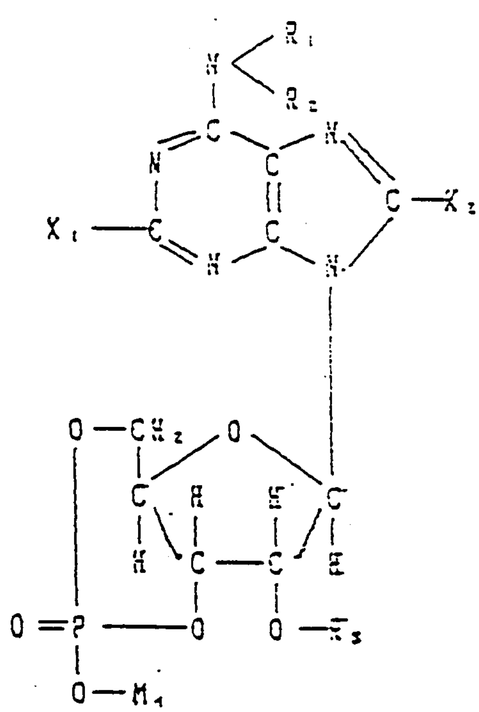

(I)

In der allgemeinen Formel (I) können $R_1$, $R_2$ und $R_3$ gleich oder unterschiedlich sein und jedes repräsentiert ein Wasserstoffatom, eine Acylgruppe mit 1 bis 25 Kohlenstoffatomen, welche ein Halogenatom aufweisen kann und welche ferner einen aromatischen Ring einschließen kann, oder eine Alkylgruppe mit 1 bis 25 Kohlenstoffatomen, welche ein Halogenatom aufweisen kann und welche ferner einen aromatischen Ring einschließen kann; $X_1$ und $X_2$ repräsentieren jeweils ein Wasserstoffatom, ein Halogen, eine Mercaptogruppe, eine Thioalkylgruppe mit 1 bis 4 Kohlenstoffatomen, eine 4-Chlorphenylthiogruppe, eine Thiobenzylgruppe, eine Aminogruppe, eine Aminoalkylgruppe mit 1 bis 4 Kohlenstoffatomen oder eine Hydroxylgruppe; und $M_1$ repräsentiert ein Wasserstoffatom, ein Alkalimetall oder Tris-(Hydroxymethyl)aminomethan.

**2.** Verwendung gemäß Anspruch 1, wobei die durch die allgemeine Formel (I) repräsentierte Verbindung Adenosin-3',5'-Cyclophosphorsäure oder dessen Salz ist.

**3.** Ein Verfahren zum Verhindern des Ergrauens des Haars oder zur Wiederherstellung von ergrautem Haar zu seiner natürlichen Farbe, welches das Anwenden einer wirksamen Menge an Lösung, die die mit Nucleinsäure verwandten Substanz nach Anspruch 1 beinhaltet, auf der Kopfhaut beinhaltet.

**4.** Ein Verfahren gemäß Anspruch 3, wobei die durch die allgemeine Formel (I) repräsentierte Verbindung Adenosin-3',5'-Cyclophosphorsäure oder dessen Salz ist.

**5.** Ein Verfahren gemäß Ansprüchen 3 und/oder 4, wobei die wirksame Menge der mit Nukleinsäure verwandten Substanzen, wobei wenigstens einer von $R_1$, $R_2$, $R_3$, $X_1$ und $X_2$ nicht Wasserstoff ist, 0,01 bis 2% beträgt.

**6.** Ein Verfahren gemäß irgendeinem der Ansprüche 3 bis 5, wobei die Zusammensetzung ein Haarwasser ist, beinhaltend 0,01 bis 2 Gew% der mit Nukleinsäure verwandten Substanz, 40 bis 98 Gew% eines niedrigeren Alkohols, 0 bis 3 Gew% pharmazeutische Mittel, 0 bis 15 Gew% eines Netzmittels, 0 bis 60 Gew% gereinigtes Wasser und eine kleine Menge eines Parfüms.

**7.** Ein Verfahren gemäß irgendeinem der Ansprüche 3 bis 5, wobei die Zusammensetzung eine Haarcreme ist, beinhaltend 0,01 bis 2 Gew% der mit Nukleinsäure verwandten Substanz, 20 bis 80 Gew% eines Öls, 0,5 bis 15 Gew% eines oberflächenaktiven Stoffes, 0 bis 15 Gew% eines Netzmittels, 15 bis 80 Gew% gereinigtes Waser und eine kleine Menge eines Konservierungsmittels.

**8.** Ein Verfahren gemäß irgendeinem der Ansprüche 3 bis 5, wobei die Zusammensetzung eine milchige Lotion ist, beinhaltend 0,01 bis 2 Gew% der mit Nukleinsäure verwandten Substanz, 5 bis 30 Gew% eines Öls, 0,5 bis 15 Gew% eines oberflächenaktiven Stoffes, 0 bis 15 Gew% eines Netzmittels, 50 bis 95 Gew% gereinigtes Wasser und eine kleine Menge eines Konservierungsmittels.

## Revendications

**1.** Utilisation d'une substance apparentée à l'acide nucléique choisie dans le groupe comprenant les composés ayant la formule générale suivante (I) et leurs sels pour la fabrication d'une composition pour la prévention du grisonnement des cheveux ou pour le rétablissement des cheveux gris dans leur couleur naturelle par application au cuir chevelu.

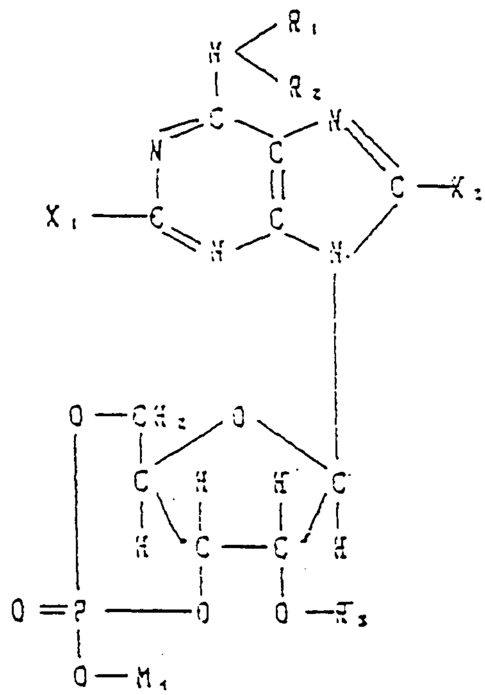

(I)

formule générale (I), dans laquelle $R_1$, $R_2$ et $R_3$ peuvent être identiques ou différents et représenter chacun un atome d'hydrogène, un groupe acyle ayant de 1 à 25 atomes de carbone, qui peut avoir un atome d'halogène et qui peut également inclure un noyau aromatique, ou un groupe alkyle ayant de 1 à 25 atomes de carbone, et peut avoir un atome d'halogène et qui peut inclure également un noyau aromatique ; $X_1$ et $X_2$ respectivement représentent un atome d'hydrogène ; un atome d'halogène, un groupe mercapto, un groupe thioalkyle ayant de 1 à 4 atomes de carbone, un groupe 4-chloro-phénylthio, un groupe thiobenzyle ; un groupe amino, un groupe aminoalkyle ayant de 1 à 4 atomes de carbone ; ou un groupe hydroxyle ; et $M_1$ représente un atome d'hydrogène, ou un cation formateur de sel.

**2.** Utilisation selon la revendication 1, selon laquelle le composé représenté par la formule (I) est l'acide adénosine phosphorique 3',5'-cyclique ou un sel de celui-ci.

**3.** Une méthode de prévention du grisonnement des cheveux ou de rétablissement des cheveux gris dans leur couleur naturelle qui comprend l'application au cuir chevelu d'une quantité efficace d'une solution comprenant la substance apparentée à l'acide nucléique selon la revendication 1.

**4.** Une méthode selon la revendication 3, selon laquelle le composé représenté par la formule générale (I) est l'acide adénosine phosphorique 3',5'-cyclique ou un sel de celui-ci.

**5.** Une méthode selon la revendication 3 et/ou la revendication 4, selon laquelle la quantité efficace de substance apparentée à l'acide nucléique dans laquelle l'un au moins des $R_1$, $R_2$, $R_3$, $X_1$ et $X_2$ n'est pas l'hydrogène est de 0,01 à 2%.

**6.** Une méthode selon l'une quelconque des revendications 3 à 5, selon laquelle la composition est un tonique capillaire comprenant 0,01 à 2% en poids de la substance apparentée à l'acide nucléique, 40 à 98% en poids d'un alcool inférieur, 0 à 3% en poids d'agents pharmaceutiques, 0 à 15% en poids d'un humectant, 0 à 60% en poids d'eau purifiée et une faible quantité de parfum.

**7.** Une méthode selon l'une quelconque des revendications 3 à 5, selon laquelle la composition est une crème capillaire comprenant 0,01 à 2% en poids de substance apparentée à l'acide nucléique, 20 à 80% en poids d'une huile, 0,5 à 15% en poids d'un surfactant, 0 à 15% en poids d'un humectant, 15 à 80% en poids d'eau purifiée et une faible quantité d'un conservateur.

8. Une méthode selon l'une quelconque des revendications 3 à 5, selon laquelle la composition est une solution de lait comprenant 0,01 à 2% en poids d'une substance apparentée à l'acide nucléique, 5 à 30% en poids d'une huile, 0,5 à 15% en poids d'un surfactant, 0 à 15% en poids d'un humectant, 50 à 95% en poids d'eau purifiée et une faible quantité d'un conservateur.